# EUROPEAN PATENT APPLICATION

(11) **EP 4 043 559 A1**
(43) Date of publication of application: **17.08.2022**
(21) Application number: 20874785.7
(22) Date of filing: 10.10.2020
(51) Int. Cl.: C12N 9/06, A61K 38/43, A61P 19/06

(54) **IMPROVED URICASE AND METHOD FOR TREATING HYPERURICEMIA USING SAME**

(30) Priority: 11.10.2019 CN 201910964325
(71) Applicant: Shanghai Junshi Biosciences Co., Ltd., Pilot Free Trade Zone Shanghai 201210 (CN); Suzhou Junmeng Biosciences Co., Ltd., Jiangsu 215002 (CN)
(72) Inventor: HUANG, Guofeng, Jiangsu 215002 (CN); ZHAO, Peng, Jiangsu 215002 (CN); CHEN, Si, Jiangsu 215002 (CN); LI, Tie, Jiangsu 215002 (CN); LI, Ruisheng, Jiangsu 215002 (CN); ZHANG, Jing, Jiangsu 215002 (CN); ZHOU, Yuehua, Jiangsu 215002 (CN); LIU, Hongchuan, Jiangsu 215002 (CN); YAO, Sheng, Jiangsu 215002 (CN)
(74) Representative: V.O.
(86) International application number: PCT/CN2020/120133
(87) International publication number: WO 2021/068925

(57) **Abstract**

Provided are an improved uricase, a method for treating hyperuricemia using the same, and a corresponding pharmaceutical composition. The improved uricase comprises an amino acid sequence having at least about 90% identity with SEQ ID NO: 1, wherein the sequence is not SEQ ID NO: 1.

SEQUENCE LISTING

<110>  Shanghai Junshi Biosciences Co., Ltd.
       Suzhou Junmeng Biosciences Co., Ltd.

<120>  IMPROVED URICASE AND METHOD FOR TREATING HYPERURICEMIA USING SAME

<130>  198134

<150>  CN 201910964325.4
<151>  2019-10-11

<160>  9

<170>  SIPOSequenceListing 1.0

<210>  1
<211>  302
<212>  PRT
<213>  Arthrobacter globiformis

<400>  1
Met Thr Ala Thr Ala Glu Thr Ser Thr Gly Thr Lys Val Val Leu Gly
1               5                   10                  15
Gln Asn Gln Tyr Gly Lys Ala Glu Val Arg Leu Val Lys Val Thr Arg
            20                  25                  30
Asn Thr Ala Arg His Glu Ile Gln Asp Leu Asn Val Thr Ser Gln Leu
        35                  40                  45
Arg Gly Asp Phe Glu Ala Ala His Thr Ala Gly Asp Asn Ala His Val
    50                  55                  60
Val Ala Thr Asp Thr Gln Lys Asn Thr Val Tyr Ala Phe Ala Arg Asp
65                  70                  75                  80
Gly Phe Ala Thr Thr Glu Glu Phe Leu Leu Arg Leu Gly Lys His Phe
                85                  90                  95
Thr Glu Gly Phe Asp Trp Val Thr Gly Gly Arg Trp Ala Ala Gln Gln
            100                 105                 110
Phe Phe Trp Asp Arg Ile Asn Asp His Asp His Ala Phe Ser Arg Asn
        115                 120                 125
Lys Ser Glu Val Arg Thr Ala Val Leu Glu Ile Ser Gly Ser Glu Gln
    130                 135                 140
Ala Ile Val Ala Gly Ile Glu Gly Leu Thr Val Leu Lys Ser Thr Gly
145                 150                 155                 160
Ser Glu Phe His Gly Phe Pro Arg Asp Lys Tyr Thr Thr Leu Gln Glu
                165                 170                 175
Thr Thr Asp Arg Ile Leu Ala Thr Asp Val Ser Ala Arg Trp Arg Tyr
            180                 185                 190
Asn Thr Val Glu Val Asp Phe Asp Ala Val Tyr Ala Ser Val Arg Gly
        195                 200                 205
Leu Leu Leu Lys Ala Phe Ala Glu Thr His Ser Leu Ala Leu Gln Gln
    210                 215                 220
Thr Met Tyr Glu Met Gly Arg Ala Val Ile Glu Thr His Pro Glu Ile
225                 230                 235                 240
Asp Glu Ile Lys Met Ser Leu Pro Asn Lys His His Phe Leu Val Asp
                245                 250                 255
Leu Gln Pro Phe Gly Gln Asp Asn Pro Asn Glu Val Phe Tyr Ala Ala
            260                 265                 270
Asp Arg Pro Tyr Gly Leu Ile Glu Ala Thr Ile Gln Arg Glu Gly Ser
        275                 280                 285
Arg Ala Asp His Pro Ile Trp Ser Asn Ile Ala Gly Phe Cys
    290                 295                 300

<210>  2
<211>  290

<212>  PRT
<213>  Artificial sequence

<220>
<223>  Amino acid sequence of MEDI4945

<400>  2
Met Ala Thr Ala Glu Thr Ser Thr Gly Cys Lys Val Val Leu Gly Gln
1               5                   10                  15
Asn Gln Tyr Gly Lys Ala Glu Val Arg Leu Val Lys Val Thr Arg Cys
            20                  25                  30
Thr Ala Arg His Glu Ile Gln Asp Leu Asn Val Thr Ser Gln Leu Ser
        35                  40                  45
Gly Asp Phe Glu Ala Ala His Thr Ala Gly Asp Asn Ala His Val Val
    50                  55                  60
Ala Thr Asp Thr Gln Lys Asn Thr Val Tyr Ala Phe Ala Arg Asp Gly
65                  70                  75                  80
Phe Ala Thr Thr Glu Glu Phe Leu Leu Arg Leu Gly Lys His Phe Thr
                85                  90                  95
Glu Gly Phe Asp Trp Val Thr Gly Gly Arg Trp Ala Ala Gln Gln Phe
            100                 105                 110
Phe Trp Asp Arg Ile Asn Asp His Asp His Ala Phe Ser Arg Asn Lys
        115                 120                 125
Ser Glu Val Arg Thr Ala Val Leu Glu Ile Ser Gly Ser Glu Gln Ala
    130                 135                 140
Ile Val Ala Gly Ile Glu Gly Leu Thr Val Leu Lys Ser Thr Gly Ser
145                 150                 155                 160
Glu Phe His Gly Phe Pro Arg Asp Lys Tyr Thr Thr Leu Gln Glu Thr
                165                 170                 175
Thr Asp Arg Ile Leu Ala Thr Asp Val Ser Ala Arg Trp Arg Tyr Asn
            180                 185                 190
Thr Val Glu Val Asp Phe Asp Ala Val Tyr Ala Ser Val Arg Gly Leu
        195                 200                 205
Leu Leu Lys Ala Phe Ala Glu Thr His Ser Leu Ala Leu Gln Gln Thr
    210                 215                 220
Met Tyr Glu Met Gly Arg Ala Val Ile Glu Thr His Pro Glu Ile Asp
225                 230                 235                 240
Glu Ile Lys Met Ser Leu Pro Asn Lys His His Phe Leu Val Asp Leu
                245                 250                 255
Gln Pro Phe Gly Gln Asp Asn Pro Asn Glu Val Phe Tyr Ala Ala Asp
            260                 265                 270
Arg Pro Tyr Gly Leu Ile Glu Ala Thr Ile Gln Arg Glu Gly Ser Arg
        275                 280                 285
Ala Asp
    290

<210>  3
<211>  297
<212>  PRT
<213>  Artificial sequence

<220>
<223>  C-terminally truncated uricase

<400>  3
Met Thr Ala Thr Ala Glu Thr Ser Thr Gly Thr Lys Val Val Leu Gly
1               5                   10                  15
Gln Asn Gln Tyr Gly Lys Ala Glu Val Arg Leu Val Lys Val Thr Arg
            20                  25                  30
Asn Thr Ala Arg His Glu Ile Gln Asp Leu Asn Val Thr Ser Gln Leu
        35                  40                  45
Arg Gly Asp Phe Glu Ala Ala His Thr Ala Gly Asp Asn Ala His Val
    50                  55                  60

Val Ala Thr Asp Thr Gln Lys Asn Thr Val Tyr Ala Phe Ala Arg Asp
65                  70                  75                  80
Gly Phe Ala Thr Thr Glu Glu Phe Leu Leu Arg Leu Gly Lys His Phe
                85                  90                  95
Thr Glu Gly Phe Asp Trp Val Thr Gly Gly Arg Trp Ala Ala Gln Gln
            100                 105                 110
Phe Phe Trp Asp Arg Ile Asn Asp His Asp His Ala Phe Ser Arg Asn
        115                 120                 125
Lys Ser Glu Val Arg Thr Ala Val Leu Glu Ile Ser Gly Ser Glu Gln
    130                 135                 140
Ala Ile Val Ala Gly Ile Glu Gly Leu Thr Val Leu Lys Ser Thr Gly
145                 150                 155                 160
Ser Glu Phe His Gly Phe Pro Arg Asp Lys Tyr Thr Thr Leu Gln Glu
                165                 170                 175
Thr Thr Asp Arg Ile Leu Ala Thr Asp Val Ser Ala Arg Trp Arg Tyr
            180                 185                 190
Asn Thr Val Glu Val Asp Phe Asp Ala Val Tyr Ala Ser Val Arg Gly
        195                 200                 205
Leu Leu Leu Lys Ala Phe Ala Glu Thr His Ser Leu Ala Leu Gln Gln
    210                 215                 220
Thr Met Tyr Glu Met Gly Arg Ala Val Ile Glu Thr His Pro Glu Ile
225                 230                 235                 240
Asp Glu Ile Lys Met Ser Leu Pro Asn Lys His His Phe Leu Val Asp
                245                 250                 255
Leu Gln Pro Phe Gly Gln Asp Asn Pro Asn Glu Val Phe Tyr Ala Ala
            260                 265                 270
Asp Arg Pro Tyr Gly Leu Ile Glu Ala Thr Ile Gln Arg Glu Gly Ser
        275                 280                 285
Arg Ala Asp His Pro Ile Trp Ser Asn
    290                 295

<210>  4
<211>  288
<212>  PRT
<213>  Artificial sequence

<220>
<223>  N-terminally amino acid-replaced uricase

<400>  4
Met Thr Asn Ile Ile Leu Gly Lys Asn Gln Tyr Gly Lys Ala Glu Val
1               5                   10                  15
Arg Leu Val Lys Val Thr Arg Asn Thr Ala Arg His Glu Ile Gln Asp
            20                  25                  30
Leu Asn Val Thr Ser Gln Leu Arg Gly Asp Phe Glu Ala Ala His Thr
        35                  40                  45
Ala Gly Asp Asn Ala His Val Val Ala Thr Asp Thr Gln Lys Asn Thr
    50                  55                  60
Val Tyr Ala Phe Ala Arg Asp Gly Phe Ala Thr Thr Glu Glu Phe Leu
65                  70                  75                  80
Leu Arg Leu Gly Lys His Phe Thr Glu Gly Phe Asp Trp Val Thr Gly
                85                  90                  95
Gly Arg Trp Ala Ala Gln Gln Phe Phe Trp Asp Arg Ile Asn Asp His
            100                 105                 110
Asp His Ala Phe Ser Arg Asn Lys Ser Glu Val Arg Thr Ala Val Leu
        115                 120                 125
Glu Ile Ser Gly Ser Glu Gln Ala Ile Val Ala Gly Ile Glu Gly Leu
    130                 135                 140
Thr Val Leu Lys Ser Thr Gly Ser Glu Phe His Gly Phe Pro Arg Asp
145                 150                 155                 160
Lys Tyr Thr Thr Leu Gln Glu Thr Thr Asp Arg Ile Leu Ala Thr Asp
                165                 170                 175
Val Ser Ala Arg Trp Arg Tyr Asn Thr Val Glu Val Asp Phe Asp Ala

                180                 185                 190
Val Tyr Ala Ser Val Arg Gly Leu Leu Leu Lys Ala Phe Ala Glu Thr
        195                 200                 205
His Ser Leu Ala Leu Gln Gln Thr Met Tyr Glu Met Gly Arg Ala Val
    210                 215                 220
Ile Glu Thr His Pro Glu Ile Asp Glu Ile Lys Met Ser Leu Pro Asn
225                 230                 235                 240
Lys His His Phe Leu Val Asp Leu Gln Pro Phe Gly Gln Asp Asn Pro
                245                 250                 255
Asn Glu Val Phe Tyr Ala Ala Asp Arg Pro Tyr Gly Leu Ile Glu Ala
            260                 265                 270
Thr Ile Gln Arg Glu Gly Ser Arg Ala Asp His Pro Ile Trp Ser Asn
        275                 280                 285

<210>  5
<211>  302
<212>  PRT
<213>  Artificial sequence

<220>
<223>  Uricase with site-directed mutations

<400>  5
Met Thr Ala Thr Ala Glu Thr Ser Thr Gly Thr Lys Val Val Leu Gly
1               5                   10                  15
Gln Asn Gln Tyr Gly Lys Ala Glu Val Arg Leu Val Lys Val Thr Arg
            20                  25                  30
Asn Thr Ala Arg His Glu Ile Gln Asp Leu Asn Val Thr Ser Gln Leu
        35                  40                  45
Arg Gly Asp Phe Glu Ala Ala His Thr Ala Gly Asp Asn Ala His Val
    50                  55                  60
Val Ala Thr Asp Thr Gln Lys Asn Thr Val Tyr Ala Phe Ala Arg Asp
65                  70                  75                  80
Gly Phe Ala Thr Thr Glu Glu Phe Leu Leu Arg Leu Gly Lys His Phe
                85                  90                  95
Thr Glu Gly Phe Asp Trp Val Thr Gly Gly Arg Trp Ala Ala Gln Gln
            100                 105                 110
Phe Phe Trp Asp Arg Ile Asn Asp His Asp His Ala Phe Ser Arg Asn
        115                 120                 125
Lys Ser Glu Val Arg Thr Ala Val Leu Glu Ile Ser Gly Ser Glu Gln
    130                 135                 140
Ala Ile Val Ala Gly Ile Glu Gly Leu Thr Val Leu Lys Ser Thr Gly
145                 150                 155                 160
Ser Glu Phe His Gly Phe Pro Arg Asp Lys Tyr Thr Thr Leu Gln Glu
                165                 170                 175
Thr Thr Asp Arg Ile Leu Ala Thr Asp Val Ser Ala Arg Trp Arg Tyr
            180                 185                 190
Asn Thr Val Glu Val Asp Phe Asp Ala Val Tyr Ala Ser Val Arg Gly
        195                 200                 205
Leu Leu Leu Lys Ala Phe Ala Glu Thr His Ser Leu Ala Leu Gln Gln
    210                 215                 220
Thr Met Tyr Glu Met Gly Arg Ala Val Ile Glu Thr His Pro Glu Ile
225                 230                 235                 240
Asp Glu Ile Lys Met Ser Leu Pro Asn Lys His His Phe Leu Val Asp
                245                 250                 255
Leu Ser Pro Phe Gly Gln Asp Asn Pro Asn Glu Val Phe Tyr Ala Ala
            260                 265                 270
Asp Arg Pro Tyr Gly Leu Ile Glu Ala Thr Ile Gln Arg Glu Gly Ser
        275                 280                 285
Arg Ala Asp His Pro Ile Trp Ser Asn Ile Ala Gly Phe Cys
    290                 295                 300

<210>  6

<211>  302
<212>  PRT
<213>  Artificial sequence

<220>
<223>  Uricase with site-directed mutations

<400>  6
Met Thr Ala Thr Ala Glu Thr Ser Thr Gly Thr Lys Val Val Leu Gly
1               5                   10                  15
Gln Asn Gln Tyr Gly Lys Ala Glu Val Arg Leu Val Lys Val Thr Arg
            20                  25                  30
Asn Thr Ala Arg His Glu Ile Gln Asp Leu Asn Val Thr Ser Gln Leu
        35                  40                  45
Arg Gly Asp Phe Glu Ala Ala His Thr Ala Gly Asp Asn Ala His Val
    50                  55                  60
Val Ala Thr Asp Thr Gln Lys Asn Thr Val Tyr Ala Phe Ala Arg Asp
65                  70                  75                  80
Gly Phe Ala Thr Thr Glu Glu Phe Leu Leu Arg Leu Gly Lys His Phe
                85                  90                  95
Thr Glu Gly Phe Asp Trp Val Thr Gly Gly Arg Trp Ala Ala Gln Gln
            100                 105                 110
Phe Phe Trp Asp Arg Ile Asn Asp His Asp His Ala Phe Ser Arg Asn
        115                 120                 125
Lys Ser Glu Val Arg Thr Ala Val Leu Glu Ile Ser Gly Ser Glu Gln
    130                 135                 140
Ala Ile Val Ala Gly Ile Glu Gly Leu Thr Val Leu Lys Ser Thr Gly
145                 150                 155                 160
Ser Glu Phe His Gly Phe Pro Arg Asp Lys Tyr Thr Thr Leu Pro Glu
                165                 170                 175
Thr Glu Asp Arg Ile Leu Ala Thr Asp Val Ser Ala Arg Trp Arg Tyr
            180                 185                 190
Asn Thr Val Glu Val Asp Phe Asp Ala Val Tyr Ala Ser Val Arg Gly
        195                 200                 205
Leu Leu Leu Lys Ala Phe Ala Glu Thr His Ser Leu Ala Leu Gln Gln
    210                 215                 220
Thr Met Tyr Glu Met Gly Arg Ala Val Ile Glu Thr His Pro Glu Ile
225                 230                 235                 240
Asp Glu Ile Lys Met Ser Leu Pro Asn Lys His His Phe Leu Val Asp
                245                 250                 255
Leu Ser Pro Phe Gly Gln Asp Asn Pro Asn Glu Val Phe Tyr Ala Ala
            260                 265                 270
Asp Arg Pro Tyr Gly Leu Ile Glu Ala Thr Ile Gln Arg Glu Gly Ser
        275                 280                 285
Arg Ala Asp His Pro Ile Trp Ser Asn Ile Ala Gly Phe Cys
    290                 295                 300

<210>  7
<211>  302
<212>  PRT
<213>  Artificial sequence

<220>
<223>  Uricase with site-directed mutations

<400>  7
Met Thr Ala Thr Ala Glu Thr Ser Thr Gly Thr Lys Val Val Leu Gly
1               5                   10                  15
Gln Asn Gln Tyr Gly Lys Ala Glu Val Arg Leu Val Lys Val Thr Arg
            20                  25                  30
Asn Thr Ala Arg His Glu Ile Gln Asp Leu Asn Val Thr Ser Gln Leu
        35                  40                  45
Arg Gly Asp Phe Glu Ala Ala His Thr Ala Gly Asp Asn Ala His Val

50                  55                  60
Val Ala Thr Asp Thr Gln Lys Asn Thr Val Tyr Ala Phe Ala Arg Asp
65                  70                  75                  80
Gly Phe Ala Thr Thr Glu Glu Phe Leu Leu Arg Leu Gly Lys His Phe
                85                  90                  95
Thr Glu Gly Phe Asp Trp Val Thr Gly Gly Arg Trp Ala Ala Gln Gln
            100                 105                 110
Phe Phe Trp Asp Arg Ile Asn Asp His Asp His Ala Phe Ser Arg Asn
        115                 120                 125
Lys Ser Glu Val Arg Thr Ala Val Leu Glu Ile Ser Gly Ser Glu Gln
    130                 135                 140
Ala Ile Val Ala Gly Ile Glu Gly Leu Thr Val Leu Lys Ser Thr Gly
145                 150                 155                 160
Ser Glu Phe His Gly Phe Pro Arg Asp Lys Tyr Thr Thr Leu Pro Glu
                165                 170                 175
Thr Glu Asp Arg Ile Leu Ala Thr Asp Val Ser Ala Arg Trp Arg Tyr
            180                 185                 190
Asn Thr Val Glu Val Asp Phe Asp Ala Val Tyr Ala Ser Val Arg Gly
        195                 200                 205
Leu Leu Leu Lys Ala Phe Ala Glu Thr His Ser Leu Ala Leu Gln Gln
    210                 215                 220
Thr Met Tyr Glu Met Gly Arg Ala Val Ile Glu Thr His Pro Glu Ile
225                 230                 235                 240
Asp Glu Ile Lys Met Ser Leu Pro Asn Lys His His Phe Leu Val Asp
                245                 250                 255
Leu Ser Pro Phe Gly Gln Asp Asn Pro Asn Glu Val Phe Tyr Ala Ala
            260                 265                 270
Asp Arg Pro Tyr Gly Leu Ile Glu Ala Thr Ile Gln Arg Glu Gly Ser
        275                 280                 285
Arg Ala Asp His Pro Ile Trp Ser Asn Ile Ala Gly Phe Cys
    290                 295                 300

<210>  8
<211>  288
<212>  PRT
<213>  Artificial sequence

<220>
<223>  N-terminally optimized uricase

<400>  8
Met Ala Asn Ile Ile Leu Gly Lys Asn Gln Tyr Gly Lys Ala Glu Val
1               5                   10                  15
Arg Leu Val Lys Val Thr Arg Asn Thr Ala Arg His Glu Ile Gln Asp
            20                  25                  30
Leu Asn Val Thr Ser Gln Leu Arg Gly Asp Phe Glu Ala Ala His Thr
        35                  40                  45
Ala Gly Asp Asn Ala His Val Val Ala Thr Asp Thr Gln Lys Asn Thr
    50                  55                  60
Val Tyr Ala Phe Ala Arg Asp Gly Phe Ala Thr Thr Glu Glu Phe Leu
65                  70                  75                  80
Leu Arg Leu Gly Lys His Phe Thr Glu Gly Phe Asp Trp Val Thr Gly
                85                  90                  95
Gly Arg Trp Ala Ala Gln Gln Phe Phe Trp Asp Arg Ile Asn Asp His
            100                 105                 110
Asp His Ala Phe Ser Arg Asn Lys Ser Glu Val Arg Thr Ala Val Leu
        115                 120                 125
Glu Ile Ser Gly Ser Glu Gln Ala Ile Val Ala Gly Ile Glu Gly Leu
    130                 135                 140
Thr Val Leu Lys Ser Thr Gly Ser Glu Phe His Gly Phe Pro Arg Asp
145                 150                 155                 160
Lys Tyr Thr Thr Leu Gln Glu Thr Thr Asp Arg Ile Leu Ala Thr Asp
                165                 170                 175

Val Ser Ala Arg Trp Arg Tyr Asn Thr Val Glu Val Asp Phe Asp Ala
            180                 185                 190
Val Tyr Ala Ser Val Arg Gly Leu Leu Leu Lys Ala Phe Ala Glu Thr
        195                 200                 205
His Ser Leu Ala Leu Gln Gln Thr Met Tyr Glu Met Gly Arg Ala Val
    210                 215                 220
Ile Glu Thr His Pro Glu Ile Asp Glu Ile Lys Met Ser Leu Pro Asn
225                 230                 235                 240
Lys His His Phe Leu Val Asp Leu Gln Pro Phe Gly Gln Asp Asn Pro
                245                 250                 255
Asn Glu Val Phe Tyr Ala Ala Asp Arg Pro Tyr Gly Leu Ile Glu Ala
            260                 265                 270
Thr Ile Gln Arg Glu Gly Ser Arg Ala Asp His Pro Ile Trp Ser Asn
        275                 280                 285

<210>  9
<211>  867
<212>  DNA
<213>  Artificial sequence

<220>
<223>  Post-codon optimization uricase coding sequence

<400>  9
atgaccgcga ccgccgaaac cagcacgggt accaaagttg ttctgggcca gaaccagtac      60
ggcaaagccg aagttcgtct ggttaaggtt acccgcaaca cgtgccgcca tgagattcaa     120
gatctgaatg tgaccagcca gctccgcggc gatttcgaag ccgcgcatac cgccggtgat     180
aatgcccatg ttgttgcgac ggacacgcag aaaaacacgg tttacgcctt cgcccgcgat     240
ggtttcgcga ccaccgaaga atttctgctc cgtctgggca aacacttcac cgaaggtttt     300
gactgggtta ccggtggtcg ttgggccgcc cagcagtttt tctgggaccg catcaacgac     360
cacgaccacg cctttagccg caataaaagc gaggtgcgta cggccgttct ggaaatcagc     420
ggtagcgaac aagccatcgt ggccggcatt gaaggtctga ccgttctgaa gagcaccggc     480
agcgaatttc atggcttccc gcgcgacaag tacacgacgc tgccagaaac cgaggatcgc     540
attctggcca ccgatgtgag tgcccgttgg cgctataaca cggtggaggt ggatttcgat     600
gccgtttacg cgagcgttcg cggtctgctg ctgaaagcgt ttgccgaaac ccatagtctg     660
gcgctgcagc agaccatgta cgaaatgggt cgcgcggtta tcgagacgca cccggaaatc     720
gacgagatca agatgagtct gccgaacaag caccactttc tggttgatct gcagccgttc     780
ggccaagata atccgaacga ggttttttac gcggccgatc gcccgtacgg tctgatcgag     840
gccaccatcc agcgcgaagg ctgctaa                                         867

## Description

### TECHNICAL FIELD

The present invention relates to an improved uricase and/or a PEG-uricase, and a method for treating hyperuricemia using the same.

### BACKGROUND

Gout and hyperuricemia are common diseases that are harmful to human health at present, and the incidence of hyperuricemia has reached 10% in the population according to the statistics. Gout is an acute arthritis caused by precipitation of urate crystals in joints due to excess uric acid in the blood. In one aspect, with changes in people's diet and lifestyle, the number of gout patients increases year by year with the intake of high-protein and high-purine food; in another aspect, after radiotherapy and chemotherapy, a large amount of uric acid will be deposited in the kidney in the short run in some patients with malignant tumors, especially leukemia and lymphoma, leading to acute kidney failure. In recent decades, many scientists have been concentrating on treating hyperuricemia-associated diseases in humans using uricases from other species. However, one of the biggest problems in applying foreign proteins to the human body is anti-protein reactions and allergic reactions caused by the immunogenicity of the proteins. Later, scientists used polyethylene glycol (PEG) to covalently modify proteins, demonstrating that it was possible to reduce protein immunogenicity, increase protein solubility and extend the half-lives of the proteins.

There are two clinically approved uricase drugs at present, Krystexxa and Elitek. Krystexxa (pegloticase) is a PEGylated uricase approved for the treatment of chronic gout in adult patients that is refractory to conventional therapy. It is a chimeric protein of the sequence of highly PEGylated porcine-baboon uricase. It can be administered within 2 hours by intravenous injection. Krystexxa contains a black box warning for anaphylaxis and infusion reactions. Elitek (rasburicase) is a modified recombinant *Aspergillus flavus* uricase indicated for the initial management of plasma uric acid levels in pediatric and adult patients with leukemia, lymphoma and solid tumor malignancies who are receiving anti-cancer therapy expected to result in tumor lysis and subsequent elevation of plasma uric acid. However, Elitek has a half-life *in vivo* of 16-21 hours. It must be administered daily by intravenous infusion. Likewise, Elitek also has a black box warning for anaphylaxis and hemolysis.

In view of the above, there is an urgent need in the art to develop drugs that are safer, more convenient, and less immunogenic for treating hyperuricemia.

### SUMMARY

In order to overcome the known side effects of uricase drugs in the prior art and obtain uricase drugs that are safer, more convenient and less immunogenic, studies are conducted according to the technical scheme of the present invention.

A first aspect of the present invention provides an improved uricase sequence, wherein the uricase comprises an amino acid sequence having at least about 90%, 91%, 92%, 93%, 94%, 95%, 96%, 97%, 98% or 99% identity with SEQ ID NO: 1, wherein the sequence is not SEQ ID NO: 1.

In some embodiments, the uricase of the present invention differs from SEQ ID NO: 1 by about 1 to about 22 amino acids (e.g., about 1, 2, 3, 4, 5, 6, 7, 8, 9, 10, 11, 12, 13, 14, 15, 16, 17, 18, 19, 20, 21 or 22 amino acids).

In some embodiments, the uricase of the present invention differs from SEQ ID NO: 1 by 22 amino acids.

In some embodiments, the uricase of the present invention differs from SEQ ID NO: 1 by 21 amino acids.

In some embodiments, the uricase of the present invention differs from SEQ ID NO: 1 by 20 amino acids.

In some embodiments, the uricase of the present invention differs from SEQ ID NO: 1 by 19 amino acids.

In some embodiments, the uricase of the present invention differs from SEQ ID NO: 1 by 18 amino acids.

In some embodiments, the uricase of the present invention differs from SEQ ID NO: 1 by 17 amino acids.

In some embodiments, the uricase of the present invention differs from SEQ ID NO: 1 by 16 amino acids.

In some embodiments, the uricase of the present invention differs from SEQ ID NO: 1 by 15 amino acids.

In some embodiments, the uricase of the present invention differs from SEQ ID NO: 1 by 14 amino acids.

In some embodiments, the uricase of the present invention differs from SEQ ID NO: 1 by 13 amino acids.

In some embodiments, the uricase of the present invention differs from SEQ ID NO: 1 by 12 amino acids.

In some embodiments, the uricase of the present invention differs from SEQ ID NO: 1 by 11 amino acids.

In some embodiments, the uricase of the present invention differs from SEQ ID NO: 1 by 10 amino acids.

In some embodiments, the uricase of the present invention differs from SEQ ID NO: 1 by 9 amino acids.

In some embodiments, the uricase of the present invention differs from SEQ ID NO: 1 by 8 amino acids.

In some embodiments, the uricase of the present invention differs from SEQ ID NO: 1 by 7 amino acids.

In some embodiments, the uricase of the present invention differs from SEQ ID NO: 1 by 6 amino acids.

In some embodiments, the uricase of the present invention differs from SEQ ID NO: 1 by 5 amino acids.

In some embodiments, the uricase of the present invention differs from SEQ ID NO: 1 by 4 amino acids.

In some embodiments, the uricase of the present invention differs from SEQ ID NO: 1 by 3 amino acids.

In some embodiments, the uricase of the present invention differs from SEQ ID NO: 1 by 2 amino acids.

In some embodiments, the uricase of the present invention differs from SEQ ID NO: 1 by 1 amino acid.

In some embodiments, the uricase of the present invention may differ from SEQ ID NO: 1 by having an amino acid sequence comprising one or more amino acid residue additions, deletions or substitutions.

In some embodiments, the uricase of the present invention is a fragment of SEQ ID NO: 1 truncated at the N-terminus and/or C-terminus, wherein the fragment of the present invention retains enzyme activity.

In some embodiments, the fragment of SEQ ID NO: 1 of the present invention is a fragment formed by truncation by 1-17 amino acids (e.g., about 1, 2, 3, 4, 5, 6, 7, 8, 9, 10, 11, 12, 13, 14, 15, 16 or 17 amino acids) at the N-terminus.

In some preferred embodiments, the uricase of the present invention has a sequence formed by truncation of SEQ ID NO: 1 by 17 amino acids at the N-terminus.

In some embodiments, the uricase of the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) and replacement with about 1-8 amino acids (e.g., about 1, 2, 3, 4, 5, 6, 7 or 8 amino acids) at the N-terminus.

In some preferred embodiments, the uricase of the present invention has a sequence formed by truncation of SEQ ID NO: 1 by 17 amino acids and replacement with about 1-8 amino acids (e.g., about 1, 2, 3, 4, 5, 6, 7 or 8 amino acids) at the N-terminus.

In some preferred embodiments, the uricase of the present invention has a sequence formed by truncation of SEQ ID NO: 1 by 17 amino acids and replacement with 8 amino acids at the N-terminus.

In some specific embodiments, the uricase of the present invention has a sequence formed by truncation of SEQ ID NO: 1 by 17 amino acids (MTATAETSTGTKVVLGQ) and replacement with amino acids MTNIILGK or MANIILGK at the N-terminus.

In some embodiments, the uricase of the present invention has a sequence formed by truncation of SEQ ID NO: 1 by about 1-5 amino acids (e.g., about 1, 2, 3, 4, or 5 amino acids) at the C-terminus.

In some preferred embodiments, the uricase of the present invention has a sequence formed by truncation of SEQ ID NO: 1 by 5 amino acids at the C-terminus.

In some embodiments, the uricase of the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) at the N-terminus and by about 1-5 amino acids (e.g., about 1, 2, 3, 4 or 5 amino acids) at the C-terminus.

In some specific embodiments, the uricase of the present invention has a sequence formed by truncation of SEQ ID NO: 1 by 17 amino acids at the N-terminus and by 5 amino acids at the C-terminus.

In some embodiments, the uricase of the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) and replacement with about 1-8 amino acids (e.g., about 1, 2, 3, 4, 5, 6, 7 or 8 amino acids) at the N-terminus, and by truncation of SEQ ID NO: 1 by about 1-5 amino acids (e.g., about 1, 2, 3, 4 or 5 amino acids) at the C-terminus.

In some preferred embodiments, the uricase of the present invention has a sequence formed by truncation of SEQ ID NO: 1 by 17 amino acids and replacement with 8 amino acids at the N-terminus, and by truncation of SEQ ID NO: 1 by 5 amino acids at the C-terminus.

In some specific embodiments, the uricase of the present invention has a sequence formed by truncation of SEQ ID NO: 1 by 17 amino acids (MTATAETSTGTKVVLGQ) and replacement with amino acids M(T/A)NIILGK at the N-terminus, and by truncation of SEQ ID NO: 1 by 5 amino acids at the C-terminus.

In another embodiment, the truncated uricase comprises about 1 to about 13 additional amino acid changes (e.g., additions, deletions or substitutions).

In some embodiments, to extend the half-life of a protein and/or reduce the immunogenicity, the uricase sequence is fusion-expressed with or covalently linked to a synthetic or biosynthetic polymer. Exemplary polymers that can be used in the present invention are polyethylene glycol (PEG), phosphorylcholine polymers, polymers of repeat peptides or "X-TEN" sequences, or carbohydrate-based polymers such as hydroxyethyl starch.

In some embodiments, the synthetic or biosynthetic polymer is fusion-expressed with the N-terminus and/or C-terminus of the uricase, so as to extend the half-life of the protein and/or reduce the immunogenicity.

In some embodiments, the uricase sequence is modified to generate 1-6 (e.g., 1, 2, 3, 4, 5 or 6) surface accessible sites for covalent linking. For example, in some embodiments, the uricase sequence is modified to contain 1, 2, 3, 4, 5, or 6 surface accessible cysteine residues, to which a polymer (e.g., PEG) may be covalently linked.

In some embodiments, by introducing new cysteine into a naturally-occurring uricase sequence at desired positions through additions, deletions and/or substitutions, site-specific covalent linking of a polymer or polypeptide capable of changing pharmacokinetic behavior is achieved.

In some embodiments, the selection of appropriate amino acids for Cys substitutions is guided by analyzing the crystal structure of uricase, so as to determine which amino acid residues are surface accessible. One or more surface accessible amino acids are then selected to be modified into cysteine.

In some embodiments, finally, these cysteines in the modified uricase sequence are located in surface accessible positions, such that these cysteines may be PEGylated.

In some embodiments, the uricase of the present invention comprises about 1 to about 7 cysteine residues, particularly about 1, 2, 3, 4, 5, 6 or 7 cysteine residues. In some embodiments, the uricase of the present invention comprises about 2 cysteine residues.

In some embodiments, the uricase of the present invention comprises a PEG moiety attached to one or more cysteine residues. Control over the number and position of cysteine residues is allowed through control over the number of PEG attachment sites and the optimal properties of the conjugate, including biophysical properties and enzyme activity.

In some embodiments, the uricase of the present invention comprises cysteine at at least one of the following positions: 35 and 194, wherein the positions are numbered relative to those in SEQ ID NO: 1.

In some embodiments, the uricase of the present invention comprises cysteine at at least one of the following positions: 35 and 194, wherein the positions are numbered relative to those in SEQ ID NO: 3.

In some embodiments, the uricase of the present invention comprises cysteine at at least one of the following positions: 35, 82, 142, 194, 216, 287 and 288, wherein the positions are numbered relative to those in SEQ ID NO: 1.

In some embodiments, the uricase of the present invention comprises cysteine at at least one of the following positions: 35, 82, 142, 194, 216, 287 and 288, wherein the positions are numbered relative to those in SEQ ID NO: 3. In some embodiments, the uricase of the present invention comprises cysteine at the following two positions: 142 and 216, 35 and 288, 35 and 142, 216 and 288, 35 and 194, or 82 and 287, wherein the positions are numbered relative to those in SEQ ID NO: 3.

In some embodiments, the uricase of the present invention comprises cysteine at the following two positions: 142 and 216, 35 and 288, 35 and 142, 216 and 288, 35 and 194, or 82 and 287, and has substitution mutations at position 205 and/or position 208, wherein the positions are numbered relative to those in SEQ ID NO: 3.

In some embodiments, the uricase of the present invention comprises cysteine at the following two positions: 142 and 216, 35 and 288, 35 and 142, 216 and 288, 35 and 194, or 82 and 287, and has an S205D or S205E substitution mutation at position 205, and/or a G208R, G208K, or G208S substitution mutation at position 208, wherein the positions are numbered relative to those in SEQ ID NO: 3.

In some embodiments, the uricase of the present invention comprises cysteine at the following two positions: 142 and 216, 35 and 288, 35 and 142, 216 and 288, 35 and 194, or 82 and 287, and has S205D and/or G208R substitution mutations, wherein the positions are numbered relative to those in SEQ ID NO: 3.

In some embodiments, the uricase of the present invention has an amino acid sequence as set forth in SEQ ID NO: 3, 4, 5, 6, 7 or 8.

In some embodiments, the uricase of the present invention has an amino acid sequence that is a variant of SEQ ID NO: 3, wherein the variant has an amino acid residue C at both positions 142 and 216, compared to SEQ ID NO: 3.

In some embodiments, the uricase of the present invention has an amino acid sequence that is a variant of SEQ ID NO: 3, wherein the variant has an amino acid residue C at both positions 35 and 288, compared to SEQ ID NO: 3.

In some embodiments, the uricase of the present invention has an amino acid sequence that is a variant of SEQ ID NO: 3, wherein the variant has an amino acid residue C at both positions 35 and 142, compared to SEQ ID NO: 3.

In some embodiments, the uricase of the present invention has an amino acid sequence that is a variant of SEQ ID NO: 3, wherein the variant has an amino acid residue C at both positions 216 and 288, compared to SEQ ID NO: 3.

In some embodiments, the uricase of the present invention has an amino acid sequence that is a variant of SEQ ID NO: 3, wherein the variant has an amino acid residue C at both positions 35 and 194, compared to SEQ ID NO: 3.

In some embodiments, the uricase of the present invention has an amino acid sequence that is a variant of SEQ ID NO: 3, wherein the variant has an amino acid residue C at both positions 82 and 287, compared to SEQ ID NO: 3.

In some embodiments, the uricase of the present invention has an amino acid sequence that is a variant of SEQ ID NO: 3, wherein the variant has an amino acid residue C at both positions 142 and 216, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 35 and 288, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 35 and 142, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 216 and 288, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 35 and 194, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 82 and 287, an amino acid residue D at position 205, and an amino acid residue R at position 208, compared to SEQ ID NO: 3.

A second aspect of the present invention provides a pharmaceutical composition comprising the uricase of the present invention, and a method for reducing uric acid levels in body fluids of a mammal using the composition of the present invention. As a preferred embodiment, the pharmaceutical composition comprises any one of the PEG-uricases mentioned above and a pharmaceutically acceptable carrier.

A third aspect of the present invention provides a method for reducing uric acid levels in body fluids and tissues of a mammal. As a preferred embodiment, the method comprises administering to the mammal an effective amount of a PEG-uricase, which is an amount that is effective in reducing uric acid levels.

### BRIEF DESCRIPTION OF THE DRAWINGS

FIG. 1 shows comparisons in specific activity between various uricases.
FIG. 2 shows thermostability experiments of various uricases.
FIG. 3 shows thermostability studies of various uricases, wherein 3a shows a PR-HPLC analysis on protein molecules at 4 °C, and 3b shows an SEC-HPLC analysis on protein molecules at 4 °C.
FIG. 4 shows an evaluation of enzyme activity kinetic studies on uricases modified by PEGylation.
FIG. 5 shows a thermostability evaluation of uricases modified by PEGylation.
FIG. 6 shows an *in vivo* efficacy evaluation of uricases modified by PEGylation.

### DETAILED DESCRIPTION

The present invention relates to an improved uricase sequence, and many different uricase sequences are encompassed herein. The uricase may comprise an amino acid sequence having at least about 90%, 91%, 92%, 93%, 94%, 95%, 96%, 97%, 98% or 99% identity with SEQ ID NO: 1, wherein the sequence of the uricase of the present invention is not SEQ ID NO: 1. The present invention also provides a PEGylation products of the improved uricase described above, and a pharmaceutical composition of the PEG-uricase described above. These improved uricases or PEG-uricases have the advantages of:
(1) having improved thermostability, solubility and enzyme activity of the protein;
(2) avoiding the high polymerization between molecules;
(3) having reduced immunogenicity and an extended half-live in the blood due to the fact that possible antigen epitopes are covered by PEG; and
(4) having enzyme activity that is similar to or higher than that of a wild-type uricase.

### Terminology

In order to facilitate the understanding of the present invention, some technical and scientific terms are specifically defined below. Unless otherwise specifically defined herein, all technical and scientific terms used herein have the same meaning as commonly understood by those of ordinary skill in the art to which the present invention belongs.

### Urate oxidase (uricase)

Urate oxidase (uricase) is a class of enzymes that catalyzes the oxidation of uric acid to more soluble allantoin. Allantoin belongs to purine metabolites, and is easier to excrete. Urate oxidase is a homotetrameric enzyme consists of four identical 34 KDa subunits, which, as an initiator for a series of reactions, converts uric acid to a more soluble and excretable product (allantoin), i.e., uricase catalyzes the reaction of uric acid (UA) with O₂ and H₂O to form the release of 5-hydroxyisourate (HIU) and H₂O₂. HIU is an unstable product that undergoes non-enzymatic hydrolysis to form 2-oxo-4-hydroxy-5-ureidoimidazole (OHCU) and then racemic allantoin after subsequent spontaneous decarboxylation. Two additional enzymes (HIU hydrolase and OHCU decarboxylase) are expressed in species with functional uricases in them. The two additional enzymes are able to catalyze these reactions more rapidly to produce (*S*)-allantoin. Functional uricases are found in a wide variety of organisms, such as archaea, bacteria, and eukaryotes. However, uricases are not expressed in humans and some primates. This is because nonsense mutations occur at codon 33 and codon 187 and a mutation occurs at a splice acceptor site in intron 2. In summary, the absence of expression of uricases in humans results in high systemic UA levels, and, in some cases, the onset of hyperuricemia, such as gout and tumor lysis syndrome. According to conservative estimation, there are up to 1.2 hundred million hyperuricemia patients in China at present. Hyperuricemia has become the fourth "hyper" disease following hypertension, hyperglycemia and hyperlipidemia. Gout is defined as an inflammatory arthritis in which serum UA levels exceed the UA solubility in the body fluids. A serum UA level above 6.8 mg/dL may cause the formation of UA crystals in tissues and thereby an acute inflammatory response. Gout causes chronic pain, dysfunctions at work and in family life, impaired health-related quality of life, etc. Tumor lysis syndrome (TLS) refers to a group of clinical syndromes in which a large amount of tumor cells are lysed in a short period, releasing intracellular metabolites, which results in hyperuricemia, hyperkalemia, hyperphosphatemia, hypocalcemia, and acute kidney failure as the main manifestations.

### Polyethylene glycol (PEG)

It is a polyether compound with the structure H-(O-CH₂-CH₂)ₙ-OH. The PEG reagents most commonly used for covalent linking of proteins are monomethoxy poly(ethylene glycol) derivatives with the structure CH₃-O-(CH₂-CH₂-O)ₙ-X, where X contains a linear linker and a reactive functional group (linear PEG), and n is an integer from 110 to 18,200. In some cases, X may contain a branched element such that a PEG reagent contains one reactive functional group and more than one PEG polymer chain (branched PEG), or more than one reactive functional group and a PEG polymer chain (forked PEG). The PEG reagent may comprise about 5, 10, 20, 40, 60 and 80 KDa of total PEG polymer.

In some embodiments, a thiol-reactive PEG may be used to react with a thiol group in at least one cysteine. For example, PEG-maleimide, as well as PEG-orthopyridyl disulfide, PEG-vinylsulfone, and PEG-iodoacetamide, can be used. In other embodiments, the thiol-reactive PEG may be a linear or branched structure with a single thiol-reactive moiety, or may be a forked structure with two or more reactive groups in each PEG molecule.

A variety of methods are known in the art to PEGylate one or more cysteines in a uricase. Any of the methods can be adopted in the present invention.

An "effective amount" includes an amount that is sufficient to ameliorate or prevent a symptom or a sign of a medical condition. An effective amount also means an amount sufficient to allow or facilitate diagnosis. The effective amount for a particular patient or veterinary object may vary depending on factors such as the condition to be treated, the general health of the patient, the route and dose of administration and the severity of side effects. An effective amount may be the maximum dose or administration regimen that avoids significant side effects or toxic effects. It may lead to an at least 5%, normally at least 10%, more normally at least 20%, most normally at least 30%, preferably at least 40%, more preferably at least 50%, most preferably at least 60%, ideally at least 70%, more ideally at least 80% and most ideally at least 90% improvement in a diagnostic measurement or parameter in the results, wherein 100% is defined as the diagnostic parameter showed by a normal subject (see, e.g., Maynard, et al., (1996) A Handbook of SOPs for Good Clinical Practice, Interpharm Press, Boca Raton, FL; Dent (2001) Good Laboratory and Good Clinical Practice, Urch Publ., London, UK).

"Identity" refers to sequence similarity between two polynucleotide sequences or between two polypeptide sequences. The identity percentage between two sequences is a function of the number of matched or identical positions shared by the two sequences divided by the number of the compared positions × 100%. For example, two sequences are 60% identical if 6 out of 10 positions of the two match or are identical when they are optimally aligned. Comparisons are typically made when two sequences are aligned to acquire the maximum identity percentage.

The "polymerase chain reaction" or "PCR" used herein generally requires that sequence information at the end of or beyond the target region must be available so that oligonucleotide primers can be designed; these primers may be identical or similar in sequence to opposite strands of the template to be amplified. The 5'-terminal nucleotide of 2 primers may coincide with the end of the amplification material. PCR can be used to amplify specific RNA sequences, specific DNA sequences from whole genomic DNA, cDNA transcribed from total cellular RNA, phage or plasmid sequences, and the like. See generally Mullis, et al., (1987) Cold Spring Harbor Symp Quant. Biol. 51: 263; Ed. Erlich (1989) PCR TECHNOLOGY (Stockton Press, N.Y). The PCR used herein is considered to be an example (but not the only one) of a nucleic acid polymerase reaction method for amplifying a nucleic acid sample, and in the method, a nucleic acid and a nucleic acid polymerase known as primers are used to amplify or generate a specific nucleic acid fragment.

### Improved uricase

The present invention relates generally to an improved uricase, PEG-uricase and use thereof for treating hyperuricemia in mammals. More specifically, the present invention provides a uricase sequence having at least about 90%, 91%, 92%, 93%, 94%, 95%, 96%, 97%, 98% or 99% identity with SEQ ID NO: 1, a PEG-uricase and use thereof, wherein the sequence is not SEQ ID NO: 1.

The uricase provided by the present invention differs from SEQ ID NO: 1 by from about 1 to about 20 amino acids (e.g., about 1, 2, 3, 4, 5, 6, 7, 8, 9, 10, 11, 12, 13, 14, 15, 16, 17, 18, 19 or 20 amino acids), i.e., has about 1 to about 20 amino acids that are different from those in SEQ ID NO: 1.

In one or more embodiments, the uricase provided by the present invention may differ from SEQ ID NO: 1 by having an amino acid sequence comprising one or more (e.g., 1-20) amino acid residue additions, deletions or substitutions. Methods for introducing amino acid additions, deletions and substitutions into an amino acid sequence are known in the art. For example, in certain embodiments, the uricase provided by the present invention has a sequence formed by truncation of SEQ ID NO: 1 at the N-terminus and/or C-terminus, wherein the truncated uricase retains enzyme activity. In certain embodiments, the uricase provided by the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) at the N-terminus; in certain embodiments, the uricase provided by the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) and replacement with about 1-8 amino acids (e.g., about 1, 2, 3, 4, 5, 6, 7 or 8 amino acids) at the N-terminus; in certain embodiments, the uricase provided by the present invention has a sequence formed by truncation of SEQ ID NO: 1 by about 1-5 amino acids (e.g., about 1, 2, 3, 4 or 5 amino acids) at the C-terminus; in certain embodiments, the uricase provided by the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) at the N-terminus and by about 5 amino acids (e.g., about 1, 2, 3, 4 or 5 amino acids) at the C-terminus; in certain embodiments, the uricase provided by the present invention has a sequence formed by truncation of SEQ ID NO: 1 by about 1-17 amino acids (e.g., about 1, 2, 3, 4, 5, 6, 7, 8, 9, 10, 11, 12, 13, 14, 15, 16 or 17 amino acids) and replacement with about 1-8 amino acids (e.g., about 1, 2, 3, 4, 5, 6, 7 or 8 amino acids) at the N-terminus, and by truncation of SEQ ID NO: 1 by about 5 amino acids (e.g., about 1, 2, 3, 4 or 5 amino acids) at the C-terminus. In some embodiments, after the truncation at the N-terminus, an amino acid sequence MTNIILGK or a fragment thereof is added as a replacement; in some embodiments, the second amino acid residue T in the sequence MTNIILGK may be optionally substituted with other amino acid residues, such as alanine or other amino acids that are non-polar amino acids as alanine is, such as proline, valine, leucine, isoleucine, phenylalanine, tryptophan and methionine.

In certain embodiments, the uricase provided by the present invention has a sequence having substitution mutations at at least one of positions 175, 178, and 258 of SEQ ID NO: 1. Preferably, the post-substitution amino acid residue at position 175 is proline or other amino acids that are non-polar amino acid as proline is, such as alanine, valine, leucine, isoleucine, phenylalanine, tryptophan and methionine; preferably, the post-substitution amino acid residue at position 178 is glutamic acid or other amino acids that are polar and charged amino acids as glutamic acid is, such as aspartic acid; preferably, the post-substitution amino acid residue at position 258 is serine or other amino acids that are polar and uncharged amino acids as serine is, such as glutamic acid, threonine, tyrosine, asparagine and glutamine. Preferably, the amino acid substitution includes at least amino acid substitutions at positions 175 and 178, optionally further an amino acid substitution at position 258. More preferably, the uricase of the present invention has a sequence of SEQ ID NO: 1 with P at position 175, E at position 178, and optionally S at position 258; further preferably or optionally, the uricase also has the truncation and/or replacement at the N-terminus and/or C-terminus as described in any one of the manners described above. Preferably, the present invention also includes an amino acid sequence having at least about 90%, 91%, 92%, 93%, 94%, 95%, 96%, 97%, 98% or 99% identity with the uricase sequence having the substitution mutations described herein, and at least the amino acid residues at positions 175, 178, and 258 in such sequences are the post-mutation residues described in the present invention, such as P, E and S, respectively. In some embodiments, the uricase provided by the present invention has a sequence further or independently having substitution mutations at position 205 and/or position 208 of SEQ ID NO: 1. In some embodiments, the uricase of the present invention has a sequence having substitution mutations at at least one of positions 205 and 208 of SEQ ID NO: 1. Preferably, the post-substitution amino acid residue at position 205 is aspartic acid or glutamic acid that is a polar and negatively charged amino acid as aspartic acid is; the post-substitution amino acid residue at position 208 is arginine or lysine or histidine that is a polar and positively charged amino acid as arginine is.

In certain embodiments, to extend the half-life of the protein and/or reduce the immunogenicity, the present invention covalently links or recombinantly expresses the uricase sequence according to any one of the embodiments herein with a synthetic or biosynthetic polymer. Exemplary polymers that can be used in the present invention are polyethylene glycol (PEG), phosphorylcholine polymers, polymers of repeat peptides or "X-TEN" sequences, or carbohydrate-based polymers such as hydroxyethyl starch. In certain embodiments, the uricase sequence provided by the present invention is modified to produce 1-6 (e.g., 1, 2, 3, 4, 5 or 6) surface accessible sites for covalent linking; for example, in some embodiments, the uricase sequence is modified to contain 1, 2, 3, 4, 5 or 6 surface accessible cysteine residues, to which a polymer (e.g., PEG) may be covalently linked; in certain embodiments, the uricase provided by the present invention comprises cysteine at at least one of the following positions: 35, 82, 142, 194, 216, 287 and 288, wherein the positions are numbered relative to those in SEQ ID NO: 1. In certain embodiments, the uricase provided by the present invention comprises cysteine at two of the following positions: 35, 82, 142, 194, 216, 287 and 288, wherein the positions are numbered relative to those in SEQ ID NO: 1 or SEQ ID NO: 3. Preferably, the uricase provided by the present invention comprises cysteine at the following two positions: 142 and 216, 35 and 288, 35 and 142, 216 and 288, 35 and 194, or 82 and 287, and particularly preferably comprises cysteine at any two positions selected from 35, 142, 194, 216 and 288. In some embodiments, the uricase with cysteine mutations is a uricase comprising cysteine at at least one of the following positions on the basis of SEQ ID NO: 1: 35, 82, 142, 194, 216, 287 and 288, wherein the positions are numbered relative to SEQ ID NO: 1. In a preferred embodiment, such uricase sequences are uricases having cysteine mutations at one or more of the positions described above based on a sequence formed by truncation of SEQ ID NO: 1 by 5 or fewer amino acid residues at the C-terminus. In some embodiments, such uricase sequences also have the substitution mutations according to any one of the embodiments above, preferably S205D and/or G208R substitution mutations, at position 205 and/or position 208, in addition to the cysteine mutations introduced as described above. In some embodiments, such uricases have a sequence formed by truncation of SEQ ID NO: 1 by 5 or fewer amino acid residues at the C-terminus, introduction of the cysteine mutations according to any one of the embodiments above (especially comprising cysteine at any two positions selected from 35, 142, 194, 216 and 288), and implementation of substitution mutations according to any one of the embodiments above at position 205 and/or position 208 (preferably S205D and/or G208R).

In some embodiments, the uricase of the present invention has an amino acid sequence that is a variant of SEQ ID NO: 3, wherein the variant has an amino acid residue C at both positions 142 and 216, compared to SEQ ID NO: 3.

In some embodiments, the uricase of the present invention has an amino acid sequence that is a variant of SEQ ID NO: 3, wherein the variant has an amino acid residue C at both positions 35 and 288, compared to SEQ ID NO: 3.

In some embodiments, the uricase of the present invention has an amino acid sequence that is a variant of SEQ ID NO: 3, wherein the variant has an amino acid residue C at both positions 35 and 142, compared to SEQ ID NO: 3.

In some embodiments, the uricase of the present invention has an amino acid sequence that is a variant of SEQ ID NO: 3, wherein the variant has an amino acid residue C at both positions 216 and 288, compared to SEQ ID NO: 3.

In some embodiments, the uricase of the present invention has an amino acid sequence that is a variant of SEQ ID NO: 3, wherein the variant has an amino acid residue C at both positions 35 and 194, compared to SEQ ID NO: 3.

In some embodiments, the uricase of the present invention has an amino acid sequence that is a variant of SEQ ID NO: 3, wherein the variant has an amino acid residue C at both positions 82 and 287, compared to SEQ ID NO: 3.

In some embodiments, the uricase of the present invention has an amino acid sequence that is a variant of SEQ ID NO: 3, wherein the variant has an amino acid residue C at both positions 142 and 216, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 35 and 288, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 35 and 142, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 216 and 288, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 35 and 194, an amino acid residue D at position 205, and an amino acid residue R at position 208, compared to SEQ ID NO: 3.

In some embodiments, the uricase of the present invention has an amino acid sequence that is a variant of SEQ ID NO: 3, wherein the variant has an amino acid residue C at both positions 82 and 287, an amino acid residue D at position 205, and an amino acid residue R at position 208, compared to SEQ ID NO: 3.

SEQ ID NO: 1 is the sequence of a wild-type *Micrococcus* uricase under the NCBI accession number D0VWQ1.1.

Methods for determining the enzymatic activity of uricases are known in the art, and any suitable method known in the art may be used to measure the enzyme activity of the uricases according to the present invention.

### Disease and Treatment or Prevention Thereof

The present invention also provides a method for treating or preventing hyperuricemia in the body fluids or tissues in a mammal with the improved uricase or PEG-uricase of the present invention. The method generally comprises administering to a subject in need thereof a therapeutically or prophylactically effective amount of the improved uricase or PEG-uricase according to any one of the embodiments of the present invention, or a pharmaceutical composition comprising the improved uricase or PEG-uricase. Suitable routes of administration may be selected as appropriate, including but not limited to oral administration, intravenous administration, subcutaneous administration, intramuscular administration, intra-arterial administration, inhalation, and aerosol delivery.

The subject typically suffers from a disease associated with elevated uric acid levels, i.e. a disease that benefits from reduction of uric acid. Typically, diseases associated with elevated uric acid levels include hyperuricemia, such as gout and tumor lysis syndrome.

### Kit

The present invention also provides a kit comprising the improved uricase or PEG-uricase of the present invention. The kit of the present invention comprises one or more components, including but not limited to the improved uricase or PEG-uricase described herein, and one or more other components, including but not limited to the pharmaceutically acceptable carrier described herein. The improved uricase or PEG-uricase may be formulated into a composition or a combination with a pharmaceutically acceptable carrier in a pharmaceutical composition.

In one embodiment, the kit comprises the improved uricase or PEG-uricase of the present invention or a pharmaceutical composition thereof in one container (e.g., a sterile glass or plastic vial).

A kit may comprise a package insert that provides information about the pharmaceutical composition and dosage form in the kit. Such information generally helps patients and physicians to use the attached pharmaceutical composition and dosage form effectively and safely. For example, the following information may be provided in the package insert regarding the combination drug disclosed herein: pharmacokinetics, pharmacodynamics, clinical studies, efficacy parameters, indications and usage, contraindications, warnings, precautions, adverse reactions, overuse, proper dosage and administration, supply specifications, proper storage conditions, references, manufacturer/wholesaler information, and patent information.

### Pharmaceutical composition and dosage

The present invention also includes pharmaceutical compositions comprising any one of the improved uricases or PEG-uricases described herein. The term "pharmaceutical composition" used herein refers to a combination of at least one drug and optionally a pharmaceutically acceptable carrier or an excipient combined together to achieve a certain objective. In some embodiments, the pharmaceutical composition includes temporally and/or spatially separated combinations, so long as they can act together to achieve the objective of the present invention. For example, the components comprised in the pharmaceutical composition (e.g., any one of the improved uricases or PEG-uricases according to the present invention) may be administered to a subject as a whole, or separately. When administered to a subject separately, the components comprised in the pharmaceutical composition may be administered to the subject simultaneously or sequentially. Preferably, the pharmaceutically acceptable carrier is water, aqueous buffer solutions, isotonic salt solutions such as PBS (phosphate buffer saline), glucose, mannitol, dextrose, lactose, starch, magnesium stearate, cellulose, magnesium carbonate, 0.3% glycerol, hyaluronic acid, ethanol, or polyalkylene glycols such as polypropylene glycol and triglyceride. The type of the pharmaceutically acceptable carrier used depends particularly on whether the composition according to the present invention is formulated for oral, nasal, intradermal, subcutaneous, intramuscular or intravenous administration. The composition according to the present invention may comprise wetting agents, emulsifying agents or buffer substances as additives.

To prepare a pharmaceutical or sterile composition of any one of the improved uricases or PEG-uricases of the present invention, the uricase or PEG-uricase can be mixed with a pharmaceutically acceptable carrier or excipient. See, e.g., *Remington's Pharmaceutical Sciences and* U.S. Pharmacopeia: National Formulary, Mack Publishing Company, Easton, PA (1984).

The pharmaceutical composition disclosed herein may be prepared in a variety of suitable dosage forms known in the art, including but not limited to lyophilized powder, ointment, aqueous solution or suspension, and the like. Therapeutic and diagnostic agents in the following dosage forms can be prepared by mixing with acceptable carriers, excipients or stabilizers: for example, lyophilized powder, ointment, aqueous solution or suspension (see, e.g., Hardman, et al., (2001) Goodman and Gilman's: The Pharmacological Basis of Therapeutics, McGraw-Hill, New York, NY; Gennaro (2000) Remington: The Science and Practice of Pharmacy, Lippincott, Williams, and Wilkins, New York, NY; Ed. Avis, et al., (1993) Pharmaceutical Dosage Forms: Parenteral Medications, Marcel Dekker, NY; Ed. Lieberman, et al. (1990) Pharmaceutical Dosage Forms: Tablets, Marcel Dekker, NY; Ed. Lieberman, et al., (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

### Use

The present invention provides use of the improved uricase or PEG-uricase according to any one of the embodiments herein in treating or preventing a disease associated with elevated uric acid levels. In certain embodiments, the present invention provides the improved uricase or PEG-uricase according to any one of the embodiments for treating or preventing a disease associated with elevated uric acid levels.

### Abbreviation

The following abbreviations are used throughout the description and examples of the present invention:
IPTG Isopropyl thiogalactopyranoside
Tm value Melting temperature
DTT Dithiothreitol

The present invention is further illustrated by the following examples, which should not be construed as limiting the present invention. The contents of all references cited throughout this application are expressly incorporated herein by reference.

### Examples

The following examples are provided to demonstrate and further illustrate some preferred embodiments and aspects of the present invention and should not be construed as limiting the scope of the present invention.

### Example 1: Preparation of Uricase

### Construction of uricase expression vector

A uricase nucleotide sequence (Suzhou Genewiz Biological Technology Co., Ltd.) was synthesized and constructed onto a PET28a expression vector. The 5' enzyme digestion site of the target gene constructed was NcoI, and the 3' enzyme digestion site was XhoI.

Taking the construction of AG-2C13 molecule as an example, the post-codon optimization nucleotide sequence was:

atgaccgcgaccgccgaaaccagcacgggtaccaaagttgttctgggccagaaccagtacggcaaagccgaagttc
gtctggttaaggttacccgcaacacgtgccgccatgagattcaagatctgaatgtgaccagccagctccgcggcgatttcgaa
gccgcgcataccgccggtgataatgcccatgttgttgcgacggacacgcagaaaaacacggtttacgccttcgcccgcgatg
gtttcgcgaccaccgaagaatttctgctccgtctgggcaaacacttcaccgaaggttttgactgggttaccggtggtcgttgggc
cgcccagcagtttttctgggaccgcatcaacgaccacgaccacgcctttagccgcaataaaagcgaggtgcgtacggccgttc
tggaaatcagcggtagcgaacaagccatcgtggccggcattgaaggtctgaccgttctgaagagcaccggcagcgaatttca
tggcttcccgcgcgacaagtacacgacgctgccagaaaccgaggatcgcattctggccaccgatgtgagtgcccgttggcgc
tataacacggtggaggtggatttcgatgccgtttacgcgagcgttcgcggtctgctgctgaaagcgtttgccgaaacccatagtc
tggcgctgcagcagaccatgtacgaaatgggtcgcgcggttatcgagacgcacccggaaatcgacgagatcaagatgagtct
gccgaacaagcaccactttctggttgatctgagcccgttcggccaagataatccgaacgaggtttttacgcggccgatcgccc
gtacggtctgatcgaggccaccatccagcgcgaaggctgctaa (SEQ ID NO: 9).

### Expression of uricase:

A transformation experiment was performed using *E. coli* BL21(DE3) (Merck), and post-transformation monoclonal colonies were picked on an LB/Kana plate. *E. coli* containing the uricase expression vector was cultured overnight at 37 °C in 5 mL of LB liquid medium (containing 50 µm/mL kanamycin), and the above seed solution was inoculated into a shake flask containing 200 mL of LB medium in an inoculation ratio of 3% and cultured at 37 °C. When the bacteria grew to OD 600 of 0.6-0.8, the culture temperature was reduced to 25 °C. IPTG was added until a final concentration of 0.3 mM was reached to induce the expression of the uricase protein of interest.

### Purification of uricase:

The bacteria were crushed by high-pressure homogenization or ultrasonication, and centrifuged. Further purification was performed by anion exchange to obtain a protein with >95% purity, which was subjected to subsequent characterization and analysis. The amino acid sequence of the protease was obtained by DNA sequencing.

Taking AG-00 as an example, the sequence was verified to be consistent with the optimized DNA sequence, and the amino acid sequence was SEQ ID NO: 1, as shown below:

MTATAETSTG TKVVLGQNQY GKAEVRLVKV TRNTARHEIQ DLNVTSQLRG DFEAAHTAGD
NAHVVATDTQ KNTVYAFARD GFATTEEFLL RLGKHFTEGF DWVTGGRWAA QQFFWDRIND
HDHAFSRNKS EVRTAVLEIS GSEQAIVAGI EGLTVLKSTG SEFHGFPRDK YTTLQETTDR
ILATDVSARW RYNTVEVDFD AVYASVRGLL LKAFAETHSL ALQQTMYEMG RAVIETHPEI
DEIKMSLPNK HHFLVDLQPF GQDNPNEVFY AADRPYGLIE ATIQREGSRA DHPIWSNIAG
FC

### Synthesis of reference uricase

A reference uricase MEDI4945 was synthesized using a method similar to that described above, having an amino acid sequence of SEQ ID NO: 2. MEDI4945 is the uricase disclosed in International Application No. PCT/US2016/032415, having an amino acid sequence shown below:

MATAETSTGCKVVLGQNQYGKAEVRLVKVTRCTARHEIQDLNVTSQLSGDFEAAHTA
GDNAHVVATDTQKNTVYAFARDGFATTEEFLLRLGKHFTEGFDWVTGGRWAAQQFF
WDRINDHDHAFSRNKSEVRTAVLEISGSEQAIVAGIEGLTVLKSTGSEFHGFPRDKYTT
LQETTDRILATDVSARWRYNTVEVDFDAVYASVRGLLLKAFAETHSLALQQTMYEMG
RAVIETHPEIDEIKMSLPNKHHFLVDLQPFGQDNPNEVFYAADRPYGLIEATIQREGSRA
D

### Example 2: Sequence Evolution of Micrococcus Uricase

### A. C-terminal optimization

SEQ ID NO: 1 was truncated by 5 amino acids at the C-terminus so as to eliminate the C-terminal Cys and thereby generate SEQ ID NO: 3. That is, a DNA specific primer was designed using a method similar to that in Example 1, and the C-terminal amino acids at positions 298-302 was deleted by PCR amplification with the AG-00 sequence as a template. The constructed product was verified by DNA sequencing, and the obtained molecule was designated AG-00-1, with the sequence shown below:

MTATAETSTG TKVVLGQNQY GKAEVRLVKV TRNTARHEIQ DLNVTSQLRG DFEAAHTAGD
NAHVVATDTQ KNTVYAFARD GFATTEEFLL RLGKHFTEGF DWVTGGRWAA QQFFWDRIND
HDHAFSRNKS EVRTAVLEIS GSEQAIVAGI EGLTVLKSTG SEFHGFPRDK YTTLQETTDR
ILATDVSARW RYNTVEVDFD AVYASVRGLL LKAFAETHSL ALQQTMYEMG RAVIETHPEI
DEIKMSLPNK HHFLVDLQPF GQDNPNEVFY AADRPYGLIE ATIQREGSRA DHPIWSN

### B. N-terminal amino acid replacement

Using a uricase database with over 15 alignments, it was found that positions 1-17 in SEQ ID NO: 1 were not highly conserved with a uricase of the same species. Therefore, a specific primer was designed using a method similar to that in Example 1, and the amino acids at positions 1-17 of the AG-00 uricase molecule were replaced with an amino acid sequence (MTNIILGK) by PCR amplification with AG-00 as a template. The constructed product was verified by DNA sequencing, showing that SEQ ID NO: 4 was generated. The molecule was designated AG-00-2, and the sequence is shown below:

MTNIILGKNQ YGKAEVRLVK VTRNTARHEI QDLNVTSQLR GDFEAAHTAG DNAHVVATDT
QKNTVYAFAR DGFATTEEFL LRLGKHFTEG FDWVTGGRWA AQQFFWDRIN DHDHAFSRNK
SEVRTAVLEI SGSEQAIVAG IEGLTVLKST GSEFHGFPRD KYTTLQETTD RILATDVSAR
WRYNTVEVDF DAVYASVRGL LLKAFAETHS LALQQTMYEM GRAVIETHPE IDEIKMSLPN
KHHFLVDLQP FGQDNPNEVF YAADRPYGLI EATIQREGSR ADHPIWSN

### C. Site-directed mutation

A DNA specific primer was designed on the basis of the AG-00 sequence using a method similar to that in Example 1, and the glutamine (Q) at position 258 was mutated into serine (S) by bridging PCR. The constructed product was verified by DNA sequencing, showing that SEQ ID NO: 5 was generated. The molecule was designated AG-01, and the sequence is shown below:

MTATAETSTG TKVVLGQNQY GKAEVRLVKV TRNTARHEIQ DLNVTSQLRG DFEAAHTAGD
NAHVVATDTQ KNTVYAFARD GFATTEEFLL RLGKHFTEGF DWVTGGRWAA QQFFWDRIND
HDHAFSRNKS EVRTAVLEIS GSEQAIVAGI EGLTVLKSTG SEFHGFPRDK YTTLQETTDR
ILATDVSARW RYNTVEVDFD AVYASVRGLL LKAFAETHSL ALQQTMYEMG RAVIETHPEI
DEIKMSLPNK HHFLVDLSPF GQDNPNEVFY AADRPYGLIE ATIQREGSRA DHPIWSNIAG
FC

In a similar way, the glutamine (Q) at position 175 was mutated into proline (P), and the threonine (T) at position 178 into glutamic acid (E). The constructed product was verified by DNA sequencing, showing that SEQ ID NO: 6 was generated. The molecule was designated AG-02, and the sequence is shown below:

MTATAETSTG TKVVLGQNQY GKAEVRLVKV TRNTARHEIQ DLNVTSQLRG DFEAAHTAGD
NAHVVATDTQ KNTVYAFARD GFATTEEFLL RLGKHFTEGF DWVTGGRWAA QQFFWDRIND
HDHAFSRNKS EVRTAVLEIS GSEQAIVAGI EGLTVLKSTG SEFHGFPRDK YTTLPETEDR
ILATDVSARW RYNTVEVDFD AVYASVRGLL LKAFAETHSL ALQQTMYEMG RAVIETHPEI
DEIKMSLPNK HHFLVDLSPF GQDNPNEVFY AADRPYGLIE ATIQREGSRA DHPIWSNIAG
FC

In a similar way, the glutamine (Q) at position 175 was mutated into proline (P), the threonine (T) at position 178 into glutamic acid (E), and the glutamine (Q) at position 258 into serine (S). The constructed product was verified by DNA sequencing, showing that SEQ ID NO: 7 was generated. The molecule was designated AG-05, and the sequence is shown below:

MTATAETSTG TKVVLGQNQY GKAEVRLVKV TRNTARHEIQ DLNVTSQLRG DFEAAHTAGD
NAHVVATDTQ KNTVYAFARD GFATTEEFLL RLGKHFTEGF DWVTGGRWAA QQFFWDRIND
HDHAFSRNKS EVRTAVLEIS GSEQAIVAGI EGLTVLKSTG SEFHGFPRDK YTTLPETEDR
ILATDVSARW RYNTVEVDFD AVYASVRGLL LKAFAETHSL ALQQTMYEMG RAVIETHPEI
DEIKMSLPNK HHFLVDLSPF GQDNPNEVFY AADRPYGLIE ATIQREGSRA DHPIWSNIAG
FC

### D. N-terminal optimization

It is known that when a protein is expressed in *E. coli,* the N-terminal methionine can be removed using Met aminopeptidase, depending on the size of the second amino acid residue after the methionine. If the second residue is small in size, then typical cleavage occurs; if the second residue is large in size, then cleavage does not occur; or if the second residue is neither large nor particularly small in size, then partial cleavage may be achieved by Met aminopeptidase, thereby leading to heterogeneity. Thus, to eliminate incomplete cleavage of the first amino acid caused by prokaryotic expression, the second threonine (T) in the amino acid sequence of SEQ ID NO: 4 was mutated into alanine (A), leading to generation of SEQ ID NO: 8. The molecule was designated AG-00-2A.

SEQ ID NO: 8

MANIILGKNQ YGKAEVRLVK VTRNTARHEI QDLNVTSQLR GDFEAAHTAG DNAHVVATDT
QKNTVYAFAR DGFATTEEFL LRLGKHFTEG FDWVTGGRWA AQQFFWDRIN DHDHAFSRNK
SEVRTAVLEI SGSEQAIVAG IEGLTVLKST GSEFHGFPRD KYTTLQETTD RILATDVSAR
WRYNTVEVDF DAVYASVRGL LLKAFAETHS LALQQTMYEM GRAVIETHPE IDEIKMSLPN
KHHFLVDLQP FGQDNPNEVF YAADRPYGLI EATIQREGSR ADHPIWSN

### E. Evaluation of enzyme activity of modified Micrococcus uricase

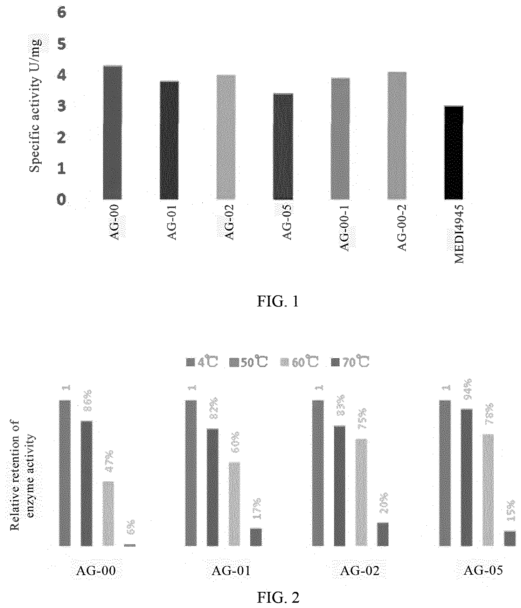

Tris-HCl buffer system (pH 8.5) was used. 0.25 mL of uric acid solution and 0.375 mL of Tris-HCl buffer were first added to a tube and incubated in a 37 °C water bath for 10 min. 0.125 mL of an appropriately diluted uricase solution (0.004 mg/mL) described above was added to the tube. After 10 min of exact reaction, 0.05 mL of 20% KOH was added to terminate the reaction. The absorbance of the residual uric acid was measured at 292 nm to evaluate the enzyme activity of modified *Micrococcus* uricase, and the results are shown in FIG. 1. It can be seen that the introduction of point mutations did not affect the enzyme activity of the uricase.

Urate oxidase activity was defined as follows: the enzyme amount required to catalyze the decomposition of 1 µmol of uric acid per minute at 37 °C and pH 8.5 was defined as 1 enzyme activity unit.

### F. Evaluation of thermostability of modified Micrococcus uricase

The above modified uricase molecule was left to stand in a water bath at 50 °C, 60 °C and 70 °C for 30 min. After 20 min of equilibration at 37 °C, 20 µL of 0.004 mg/mL uricase solution was added, and reading was immediately performed with a microplate reader. The reaction was performed for 10 min. The residual enzyme activity of the uricase was measured and expressed as difference in absorbance. Normalization was performed with the uricase enzyme activity at 4 °C, and the results are shown in FIG. 2. Activity assay system: 50 µL of 800 µM uric acid, 30 µL of buffer.

After AG-00 was subjected to heat treatment at 60 °C for half an hour, the retention of enzyme activity was 47%, whereas for AG-01, AG-02 and AG-05, the retention rates of enzyme activity were 60%, 75% and 78%, respectively, after treatment under the same conditions. After treatment at 70 °C, the retention of enzyme activity was 6% for AG-00, and the retention rates of enzyme activity for uricases AG-01, AG-02 and AG-05 were 17%, 20% and 15%, respectively. As can be seen, the introduction of point mutations improves the thermostability of enzyme activity.

### G. Evaluation of Tm value of modified Micrococcus uricase

The Tm value of the protein molecule was evaluated using the protein Thermo Shift assay kit. The uricase above was diluted to 0.5715 mg/mL with a buffer (2.34 g/L Na₂HPO₄; 0.48 g/L NaH₂PO₄·H₂O; 75.31 g/L sucrose; 0.2 g/L Tween80; pH 7.6±2); the 1000× fluorescent dye in the kit was diluted with ultrapure water to 8× fluorescent dye; 70 µL of 0.5715 mg/mL uricase was well mixed with 10 µL of 8× fluorescent dye in a 1.5 mL EP tube, and the mixture was aliquoted to an 8-tube PCR strip or 96-well PCR plate at a volume of 30 µL, for a total of 2 wells. A blank control sample was prepared by mixing 70 µL of formulation buffer with 10 µL of 8× fluorescent dye, and aliquoted to an 8-tube PCR strip or 96-well PCR plate at a volume of 30 µL, for a total of 2 wells. In addition, a system blank control was prepared by mixing 70 µL of ultrapure water with 10 µL of 8× fluorescent dye, and aliquoted to an 8-tube PCR strip or 96-well PCR plate at a volume of 30 µL, for a total of 2 wells.

| | |
|---|---|
| Measurement mode | Melting curve |
| Detection of fluorophore | ROX |
| Sample volume in each tube/well | 30 µL |
| Time for 25 °C equilibration stage | 2 min |
| Rate for warming stage | 1% |
| Time for 99 °C equilibration stage | 2 min |

The results are shown in Table 1 below.

As can be seen, the Tm D values (temperature for first protein denaturation) of AG-01, AG-02, AG-05 and AG-00-2 are all higher than those of the wild-type uricase molecule AG-00 and the reference MEDI4945. As can be seen, the introduction of point mutations improves the thermostability of uricase.

**Table 1**

| No. | Sample name | Tm D (°C) |
|---|---|---|
| 1 | MEDI4945 | 53.45 |
| 2 | AG-00 | 58.12 |
| 3 | AG-00-1 | 56.59 |
| 4 | AG-00-2 | 61.91 |
| 5 | AG-01 | 61.40 |
| 6 | AG-02 | 58.92 |
| 7 | AG-05 | 60.38 |

### Example 3: Specific PEGylation

Polyethylene glycol (PEG)-modified therapeutic proteins can be randomly attached to selected protein residues (such as lysine side chains) or are specific for particular sites. PEGylation specific for particular sites can be better controlled and result in a highly homogeneous PEGylated product with definite bioactivity. Among methods for site-specific attachment, conjugation with an unpaired cysteine residue is most widely used. The conjugation requires the introduction of one or more free cysteine residues in the protein sequence. The C-terminally truncated AG-00-1 molecular sequence (SEQ ID NO: 3) does not contain cysteine. Cysteine can be introduced by amino acid mutation.

### Introduction of Cys mutation

To select a potential site for introduction of a Cys residue in the AG-00-1 sequence, several factors were considered:
(1) the site must be far from the enzymatic active site to avoid the risk of affecting activity;
(2) sites are not close to each other to avoid steric hindrance that makes it difficult to PEGylate adjacent Cys;
(3) the site must be on the protein surface to ensure effective reaction with the thiol-reactive PEG reagent; and
(4) the site must not be in structural or conservative regions.

After analysis, sites that satisfy the above requirements included A35, F82, S142, T194, E216, G287 and S288. Accordingly, the above sites were selected for Cys mutaions.

By using a method similar to that in Example 1 to introduce Cys mutations, the following molecules were prepared: AG-2C12, AG-2C13, AG-2C16, AG-2C17, AG-1C13-1, AG-2C01, AG-2C05-1, AG-2C01-1 and AG-2C12-1.

### Protein stability study

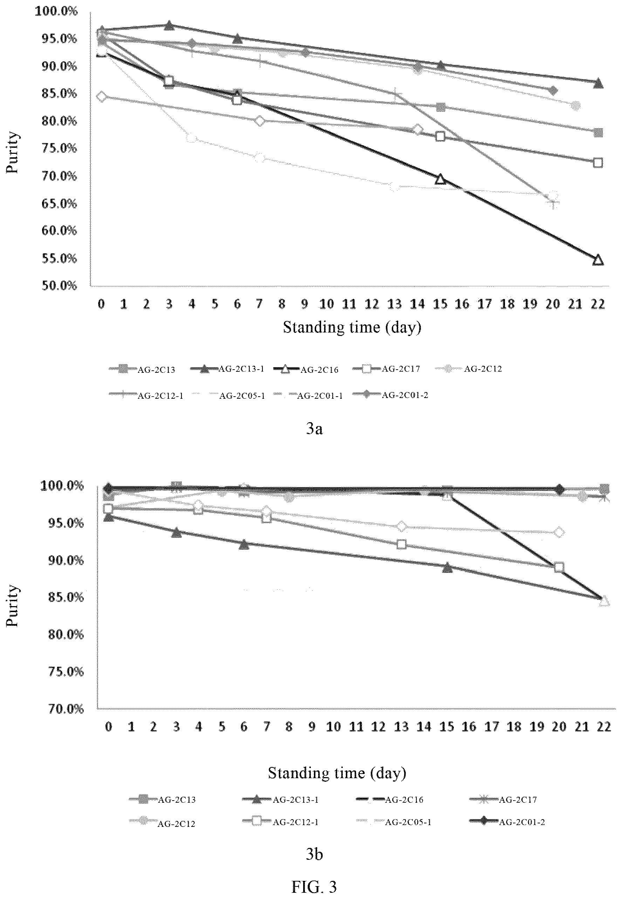

Various modified uricases were left to stand in a phosphate buffer under the conditions of pH 7.0, 1 mM DTT and 4 °C. Comparisons were made between proteins in storage stability by SEC-HPLC and RP-HPLC, and the results are shown in FIGs. 3a and 3b.

The RP-HPLC (FIG. 3a) results show that AG-2C13, AG-2C13-1, AG-2C12 and AG-2C01-2 have better purity. The SEC-HPLC (FIG. 3b) results show that AG-2C13, AG-2C17, AG-2C12 and AG-2C01-2 have better purity. According to the combination of the SEC-HPLC and RP-HPLC results, AG-2C13, AG-2C12 and AG-2C01-2 molecules were preferred.

### PEG-modification method for uricase:

To 2 mg of a uricase (100 mM PBS buffer, pH = 7.0, 150 mM NaCl) were sequentially added TCEP.HCl (0.5 mg, tris(2-carboxyethyl)phosphine hydrochloride) and M-MAL-PEG-10K (27.5 mg, maleimide polyethylene glycol, molecular weight of 10,000 Da). The mixture was allowed to react at room temperature for 2 h. The reaction mixture was purified by ion-exchange chromatography to obtain a PEGylated uricase.

Activity determination method for uricase: a phosphate buffer system (pH 7.3) was used. With reference to the above method (Example 2) for an activity assay system, the absorbance of the residual uric acid was measured at 292 nm.

The Cys mutation sites selected above were paired for PEGylation modification and uricase activity evaluation. The results are shown in Table 2.

**Table 2**

| Molecular name | Site mutation | Number of single subunits | Retention of enzyme activity | Post-modification enzyme activity U/mg |
|---|---|---|---|---|
| AG-2C12 | S142C/E216C | 2 | 98% | 2.0 |
| AG-2C13 | A35C/S288C | 2 | 90% | 1.95 |
| AG-2C16 | A35C/S142C | 2 | 98% | 1.69 |
| AG-2C17 | E216C/S288C | 2 | 90% | 1.85 |
| AG-2C13-1 | A35C/S288C | 2 | 113% | 2.33 |
| AG-2C01-2 | A35C/T194C | 2 | 99% | 1.55 |
| AG-2C05-1 | F82C/G287C | 2 | 44% | 0.67 |
| AG-2C01-1 | A35C/T194C | 2 | 92% | 1.43 |
| AG-2C12-1 | S142C/E216C | 2 | 102% | 1.65 |

| | | | | |
|---|---|---|---|---|
| ^{∗}Note: Compared to AG-2C13, AG-2C01-2 and AG-2C12, AG-2C13-1, AG-2C01-1 and AG-2C12-1 each further comprise the following mutations: S205D and G208R. | | | | |

Both cysteines introduced in the molecules listed in Table 2 can be well modified by PEG, demonstrating that the introduction sites were exposed on the molecule surface. After the single subunits of uricase were doubly modified by mPEG, the retention rate of enzyme activity varied from 44% to 113%. In addition, the introduction of mutations at positions 205 and 208 can further increase the enzyme activity.

### Example 4: Assessment of PEGylation-Modified Uricase Activity

### A. Enzyme activity kinetic study of PEGylated urokinase

Tris-HCl buffer system (pH 8.5) was used. The stock solution for uricase working solutions was 2 mM uric acid, which was stored at 4 °C. Process of enzyme activity assay:
The temperature was set at 37 °C.

Substrate (uric acid) concentration gradients: 600 µmol, 500 µmol, 400 µmol, 200 µmol, 100 µmol, 50 µmol, 25 µmol, and 12.5 µmol.

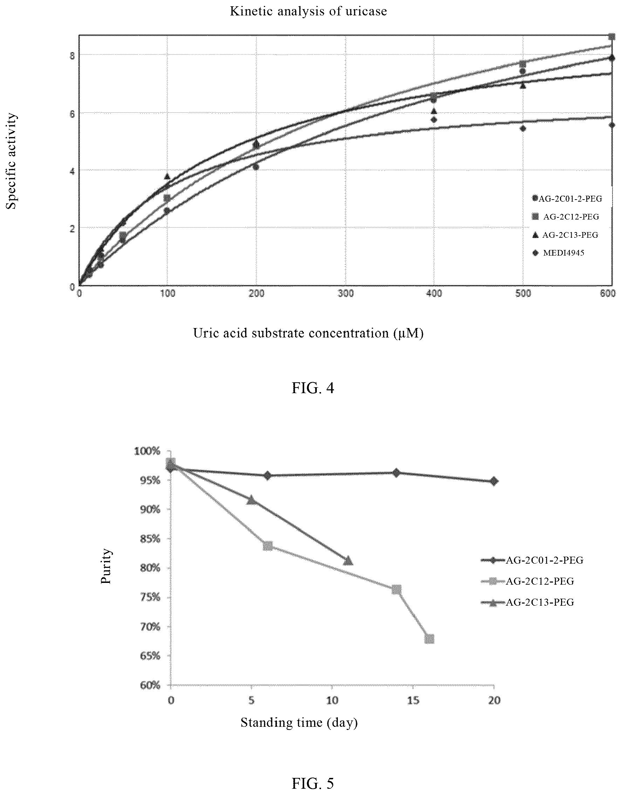

50 µL of the above uric acid solution and 30 µL of the above Tris-HCl buffer were first added to a 96-well plate and incubated at 37 °C for 20 min. Then 20 µL of an appropriately diluted uricase solution (0.004 mg/mL) was rapidly added to the 96-well plate. Procedure setting: reaction for 10 min, one data reading every 20 s, and initiation of reaction. The results are shown in FIG. 4 and Table 3.

The results show that the uricase molecules are in the following order when sorted in descending order according to their maximum reaction rates: AG-2C01-2-PEG, AG-2C12-PEG, AG-2C13-PEG and MEDI4945.

**Table 3**

| Molecular name | Km | Vmax | Kcat |
|---|---|---|---|
| AG-2C01-2-PEG | 451.4 | 13.84 | 5.4 |
| AG-2C12-PEG | 355.5 | 13.24 | 5.3 |
| AG-2C13-PEG | 167.7 | 9.4 | 3.6 |
| MEDI4945 | 101.5 | 6.8 | 2.6 |

| | | | |
|---|---|---|---|
| Note: Km: Michaelis constant; Vmax: maximum reaction rate; Kcat: turnover number, refers to the number of substrate that a single enzyme molecule converts within one second. | | | |

### B. Accelerated stability evaluation of PEG-modified urokinase (37 °C)

The PEG-modified uricase was left to stand in a 37 °C incubator. The buffer system was a phosphate buffer system at pH 7.0. The SEC-HPLC results are shown in FIG. 5. As can be seen, AG-2C01-2-PEG has superior stability to that of AG-2C12-PEG and AG-2C13-PEG at 37 °C.

### Example 6: In Vivo Pharmacodynamic Study

The effect of the test substance on serum uric acid levels was evaluated by single intravenous injection (i.v.) in a mouse model of hyperuricemia induced by intraperitoneal injection (i.p.) of exogenous uric acid. 120 normal mice (ICR-male) were randomly divided into 6 groups of 20 mice. Group 1 and group 2 were given solvent as a blank control and a solvent control, respectively. Group 3 was given MEDI4945 at a dose of 0.1 mg/kg as a positive control. Groups 4, 5 and 6 were given AG-2C13-PEG, AG-2C01-2-PEG and AG-2C12-PEG, respectively, at a dose of 0.1 mg/kg as treatment groups. On the next day after administration, except for the blank control group, the experimental mice in each group were subjected to intraperitoneal injection of uric acid for modeling (250 mg/kg, 10 mL/kg). Blood samples of animals 1-10 in each group were taken 30 min and 3 h after the modeling, and blood samples of animals 11-20 were taken 1.5 h and 6 h after the modeling.

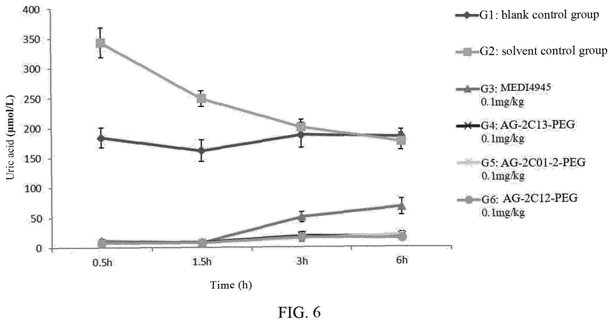

The results are shown in FIG. 6. The serum uric acid levels of the animals in the AG-2C13-PEG, AG-2C01-2-PEG and AG-2C12-PEG treatment groups at the dose of 0.1 mg/kg are significantly lower than those of the animals in the solvent control group (p < 0.01) at each time point from 0.5 h to 6 h after the modeling, and the serum uric acid levels of the animals in the 3 treatment groups are all lower than the lower limit of detection 0.5-1.5 h after the modeling (except the uric acid level of the AG-2C13-PEG group was 8.35 µmol/L at 1.5 h). At the test dose (0.1 mg/kg), after single injection of AG-2C13-PEG, AG-2C01-2-PEG and AG-2C12-PEG, the serum uric acid levels are remarkably reduced in the ICR mouse model of hyperuricemia induced by intraperitoneal injection of exogenous uric acid. At this dose, there is no significant difference in effect between urokinases AG-2C13-PEG, AG-2C01-2-PEG and AG-2C12-PEG at each time point after the modeling, but the efficacy is better than that of MEDI4945 after 3 h.

## Claims

1. A uricase comprising an amino acid sequence having at least about 90%, 91%, 92%, 93%, 94%, 95%, 96%, 97%, 98% or 99% identity with SEQ ID NO: 1, wherein the sequence is not SEQ ID NO: 1.

2. The uricase according to claim 1, wherein the uricase has an amino acid sequence comprising 1, 2, 3, 4, 5, 6, 7, 8, 9, 10, 11, 12, 13, 14, 15, 16, 17, 18, 19, 20, 21, or 22 amino acid residue additions, deletions, or substitutions compared to the sequence of SEQ ID NO: 1.

3. The uricase according to claim 2, wherein the uricase is a fragment of SEQ ID NO: 1 truncated at the N-terminus and/or the C-terminus, wherein the fragment retains enzyme activity;
preferably, the truncation is truncation by 17 or fewer amino acid residues at the N-terminus, and/or 5 or fewer amino acid residues at the C-terminus, wherein optionally, 8 or fewer amino acid residues are added after the truncation at the N-terminus.

4. The uricase according to claim 3, wherein
the truncation is truncation by 17 amino acids, i.e., MTATAETSTGTKVVLGQ, at the N-terminus;
the addition is addition of an amino acid sequence MTNIILGK or MANIILGK; or
the truncation is truncation by 5 amino acids, i.e., IAGFC, at the C-terminus.

5. The uricase according to claim 1, wherein compared to SEQ ID NO: 1, the uricase has an amino acid sequence having substitution mutations at at least one position corresponding to positions 175, 178 and 258 of SEQ ID NO: 1;
preferably, the post-substitution amino acid residue at position 175 is proline, alanine, valine, leucine, isoleucine, phenylalanine, tryptophan, or methionine;
preferably, the post-substitution amino acid residue at position 178 is glutamic acid or aspartic acid;
preferably, the post-substitution amino acid residue at position 258 is serine, glutamic acid, threonine, tyrosine, asparagine, or glutamine.

6. The uricase according to any one of claims 1-5, wherein the sequence of the uricase has 1-6 surface accessible cysteine residues introduced by mutation, and optionally has substitution mutations at position 205 and/or position 208, wherein the positions are numbered relative to those in SEQ ID NO: 1 or 3.

7. The uricase according to claim 6, wherein the uricase comprises cysteine residues at 1-6 of the following positions: 35, 82, 142, 194, 216, 287 and 288, wherein the positions are numbered relative to those in SEQ ID NO: 1 or 3;
preferably, the uricase comprises cysteine residues at two of the following positions: 35, 82, 142, 194, 216, 287 and 288, wherein the positions are numbered relative to those in SEQ ID NO: 1 or 3;
more preferably, the uricase comprises cysteine residues at the following two positions: 142 and 216, 35 and 288, 35 and 142, 216 and 288, 35 and 194, or 82 and 287, wherein the positions are numbered relative to those in SEQ ID NO: 1 or 3.

8. The uricase according to claim 1, wherein the uricase has an amino acid sequence as set forth in SEQ ID NO: 3, 4, 5, 6, 7 or 8, or has an amino acid sequence comprising cysteine at the following two positions of the sequence of SEQ ID NO: 3: 142 and 216, 35 and 288, 35 and 142, 216 and 288, 35 and 194, or 82 and 287, and optionally having substitution mutations at position 205 and/or position 208 of SEQ ID NO: 3, wherein preferably, the substitution mutations are a substitution of D or E for S at position 205 and a substitution of R, K or S for G at position 208 of SEQ ID NO: 3.

9. The uricase according to claim 8, wherein the uricase has an amino acid sequence comprising cysteine at the following two positions of the sequence of SEQ ID NO: 3: 142 and 216, 35 and 288, 35 and 142, 216 and 288, 35 and 194, or 82 and 287, and optionally comprising S205D and/or G208R substitution mutations.

10. The uricase according to claim 9, wherein the uricase has an amino acid sequence that is:
a variant of SEQ ID NO: 3, wherein the variant has an amino acid residue C at both positions 142 and 216, compared to SEQ ID NO: 3; or
a variant of SEQ ID NO: 3, wherein the variant has an amino acid residue C at both positions 35 and 288, compared to SEQ ID NO: 3; or
a variant of SEQ ID NO: 3, wherein the variant has an amino acid residue C at both positions 35 and 142, compared to SEQ ID NO: 3; or
a variant of SEQ ID NO: 3, wherein the variant has an amino acid residue C at both positions 216 and 288, compared to SEQ ID NO: 3; or
a variant of SEQ ID NO: 3, wherein the variant has an amino acid residue C at both positions 35 and 194, compared to SEQ ID NO: 3; or
a variant of SEQ ID NO: 3, wherein the variant has an amino acid residue C at both positions 82 and 287, compared to SEQ ID NO: 3; or
a variant of SEQ ID NO: 3, wherein the variant has an amino acid residue C at both positions 142 and 216, an amino acid residue D at position 205, and an amino acid residue R at position 208, compared to SEQ ID NO: 3; or
a variant of SEQ ID NO: 3, wherein the variant has an amino acid residue C at both positions 35 and 288, an amino acid residue D at position 205, and an amino acid residue R at position 208, compared to SEQ ID NO: 3; or
a variant of SEQ ID NO: 3, wherein the variant has an amino acid residue C at both positions 35 and 142, an amino acid residue D at position 205, and an amino acid residue R at position 208, compared to SEQ ID NO: 3; or
a variant of SEQ ID NO: 3, wherein the variant has an amino acid residue C at both positions 216 and 288, an amino acid residue D at position 205, and an amino acid residue R at position 208, compared to SEQ ID NO: 3; or
a variant of SEQ ID NO: 3, wherein the variant has an amino acid residue C at both positions 35 and 194, an amino acid residue D at position 205, and an amino acid residue R at position 208, compared to SEQ ID NO: 3; or
a variant of SEQ ID NO: 3, wherein the variant has an amino acid residue C at both positions 82 and 287, an amino acid residue D at position 205, and an amino acid residue R at position 208, compared to SEQ ID NO: 3.

11. A conjugate comprising the uricase according to any one of claims 1-10 and a synthetic polymer covalently linked to or fusion-expressed with the uricase.

12. The conjugate according to claim 11, wherein the polymer is selected from polyethylene glycol (PEG), phosphorylcholine polymers, polymers of repeat peptides or "X-TEN" sequences, and carbohydrate-based polymers.

13. The conjugate according to claim 12, wherein the polymer is fusion-expressed with the N-terminus and/or C-terminus of the uricase, or is covalently linked through a cysteine residue present on the uricase.

14. A pharmaceutical composition comprising the uricase according to any one of claims 1-10 or the conjugate according to any one of claims 11-13, and a pharmaceutically acceptable carrier.

15. Use of the uricase according to any one of claims 1-10, the conjugate according to any one of claims 11-13, or the pharmaceutical composition according to claim 14 in preparing a medicament for reducing uric acid levels in body fluids and tissues of a mammal or treating hyperuricemia.
